(19) Europäisches Patentamt
European Patent Office
Office européen des brevets

(11) EP 1 153 916 A2

(12) **EUROPÄISCHE PATENTANMELDUNG**

(43) Veröffentlichungstag:
**14.11.2001 Patentblatt 2001/46**

(51) Int Cl.[7]: **C07C 323/29**, C07C 323/30, C08C 19/30

(21) Anmeldenummer: **01110081.5**

(22) Anmeldetag: **30.04.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **11.05.2000 DE 10022950**

(71) Anmelder: **BAYER AG**
**51368 Leverkusen (DE)**

(72) Erfinder:
- **Laue, Christian, Dr.**
  **40789 Monheim (DE)**
- **Oberthür, Markus, Dr.**
  **41540 Dormagen (DE)**

(54) **Covulkanisierbare Alterungsschutzmittel**

(57) Die Erfindung betrifft covulkanisierbare Alterungsschutzmittel, herstellbar durch Umsetzung von gegebenenfalls substituierten p-Phenylendiaminen und/oder sterisch gehinderten Phenolen mit bifunktionellen Alkyl-, Aryl- und/oder Aralkylverbindungen und anschließender Reaktion des so erhaltenen Produkts mit Schwefel und/oder schwefelliefernden Verbindungen. Die erfindungsgemäßen Alterungsschutzmittel erhalten ihre Wirksamkeit über einen langen Zeitraum und zeichnen sich vor allem dadurch aus, dass sie praktisch kaum durch Wasser, Öle und/oder Benzine oder Hydraulikflüssigkeiten aus den Vulkanisaten extrahiert werden.

EP 1 153 916 A2

## Beschreibung

Die vorliegende Erfindung betrifft covulkanisierbare Alterungsschutzmittel, die Kautschukvulkanisate langfristig gegen thermische Alterung, Ermüdung sowie gegen Alterung durch Sauerstoffeinfluss zu schützen vermögen. Die erfindungsgemäßen Alterungsschutzmittel zeichnen sich weiterhin dadurch aus, dass sie praktisch kaum durch Wasser, Öle und/oder Benzine oder Hydraulikflüssigkeiten aus den Vulkanisaten extrahiert werden.

Es ist bekannt, Kautschukvulkanisate durch Alterungsschutzmittel gegen die Vulkanisate zerstörende Umwelteinflüsse zu schützen. So werden z.B. zur Verbesserung der Hitze- und Lagerstabilität von Kautschukvulkanisaten bekannte phenolische, aminische, schwefelhaltige oder phosphorhaltige Alterungsschutzmittel zugegeben. Diese sind z.B. in Ullmanns Enzyklopädie der technischen Chemie, Band 8, S. 19 ff näher beschrieben.

Darüber hinaus ist es bekannt, die Flüchtigkeit der Alterungsschutzmittel dadurch zu verringern, dass sie beispielsweise auf Trägermaterialien aufgebracht sind und/oder mit reaktiven Gruppen versehen werden, um bei der Herstellung der Kautschuke copolymerisiert zu werden, oder dass sie vor der Vulkanisation auf den zu schützenden Kautschuk durch Pfropfung aufgebracht werden. Die auf diese Weise modifizierten Alterungsschutzmittel sind beispielsweise beschrieben in JP 61 111 343, W. W. Schunk, Gummi, Fasern, Kunstst. 43 (3), (1990), 138-144, R. H. Kline, J. P. Miller, Rubber Chem. Technol. 46 (1), (1973), 96-105, EP-A 466 263, DE-A 19 718 288, EP-A 120 801, H. Fries, Gummi, Asbest, Kunstst. 40, (1987), 238-258, D. Braun, R. Rettig, W. Rogler, Angew. Makromol. Chem. 211, (1993), 165-194, DE-A 3 430 510, G. Scott, S. M. Tavakoli, Polym. Degrad. Stab. 4 (4), (1982), 279-285, A. H. Weinstein, Rubber Chem. Technol. 50 (4), (1977), 650-659.

Nachteilig bei den bislang bekannten Alterungsschutzmitteln für Kautschukvulkanisate ist zum einen deren Flüchtigkeit oder Extrahierbarkeit zum anderen, insbesondere bei den modifizierten Alterungsschutzmitteln, dass sie schon bei der Polymerisation der Monomeren zur Herstellung der Kautschuke zugegen sind und daher die Polymerisationsreaktion ungünstig beeinflussen (z.B. Verringerung der Reaktionsgeschwindigkeit).

Es ist somit Aufgabe der vorliegenden Erfindung, ein Alterungsschutzmittel zur Verfügung zu stellen, das zum einen die Polymerisation der Monomeren nicht ungünstig beeinflusst und zum anderen die Flüchtigkeit und Extrahierbarkeit der bekannten Alterungsschutzmittel vermeidet, ohne dass die Wirksamkeit des erfindungsgemäßen Alterungsschutzmittels gegenüber den bisher bekannten Alterungsschutzmitteln vermindert ist.

Gegenstand der vorliegenden Erfindung sind nun covulkanisierbare Alterungsschutzmittel, herstellbar durch Umsetzung von zunächst gegebenenfalls substituierten p-Phenylendiaminen und/oder sterisch gehinderten Phenolen mit bifunktionellen Alkyl-, Aryl- und/oder Aralkylverbindungen und anschließender Reaktion des so erhaltenen Produktes mit Schwefel und/oder schwefelliefernden Verbindungen.

Als gegebenenfalls substituierte p-Phenylendiamine kommen solche der Formel (I)

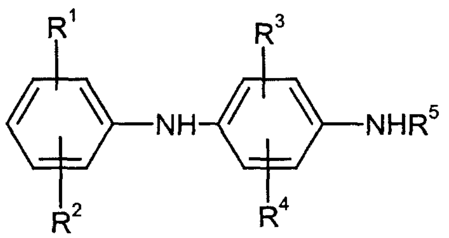

(I)

in Frage, in der

$R^1$ bis $R^4$ gleich oder verschieden sind und für Wasserstoff, geradkettiges oder verzweigtes $C_1$-$C_{12}$-Alkyl, $C_1$-$C_{12}$-Alkoxy, $C_1$-$C_{12}$-Alkyl-thio, $C_1$-$C_{12}$-Alkyl-amino, Di-($C_1$-$C_{12}$-alkyl)-amino, Benzyl, 1,1-Dimethylbenzyl oder Phenyl stehen
und

$R^5$ für Wasserstoff, Phenyl, $C_6$-$C_{12}$-Aryl, $C_1$-$C_{12}$-Heteroaryl oder $C_1$-$C_{12}$-Alkyl steht.

Unter $C_1$-$C_{12}$-Alkyl werden sämtliche dem Fachmann bekannte lineare, cyclische oder verzweigte Alkylreste mit 1 bis 12 C-Atomen verstanden, wie Methyl, Ethyl, n-Propyl, i-Propyl, n-Butyl, t-Butyl, n-Pentyl, i-Pentyl, neo-Pentyl, n-Hexyl, Cyclohexyl, i-Hexyl, Heptyl, Octyl, Nonyl, Decyl, Undecyl und Dodecyl, die ihrerseits wiederum substituiert sein können.

Als Substituenten kommen hierbei Halogen, Nitro, Hydroxyl oder auch $C_1$-$C_{12}$-Alkyl, $C_1$-$C_{12}$-Alkoxy, $C_5$-$C_{12}$-Cycloalkyl, $C_6$-$C_{12}$-Aryl, $C_1$-$C_{12}$-Heteroaryl in Frage, wie Benzyl, Trimethylphenyl, Ethylphenyl, Chlormethyl, Chlorethyl oder Nitromethyl.

Unter $C_1$-$C_{12}$-Alkoxyl werden sämtliche dem Fachmann bekannte lineare oder verzweigte Alkoxylreste mit 1 bis 12 C-Atomen verstanden, wie Methoxy, Ethoxy, n-Propoxy, i-Propoxy, n-Butoxy, i-Butoxy, t-Butoxy, n-Pentoxy, i-Pentoxy, neo-Pentoxy und Hexoxy, die ihrerseits wiederum durch die obengenannten Substituenten substituiert sein können.

Unter $C_5$-$C_{12}$-Cycloalkyl werden sämtliche dem Fachmann bekannte ein- oder mehrkernige Cycloalkylreste mit 5 bis 12 C-Atomen verstanden, wie Cyclopentyl, Cyclohexyl, Cycloheptyl, Cyclooctyl und Cyclononyl, die ihrerseits wiederum durch die oben genannten Substituenten substituiert sein können.

Unter $C_6$-$C_{12}$-Aryl werden sämtliche dem Fachmann bekannte ein- oder mehrkernige Arylreste mit 6 bis 12 C-Atomen verstanden, wie Phenyl, Naphthyl, die ihrerseits wiederum durch die obengenannten Substituenten substituiert sein können.

Unter $C_1$-$C_{12}$-Heteroaryl werden sämtliche dem Fachmann bekannten ein- oder mehrkernige Heteroarylreste verstanden, die neben 1 bis 12 C-Atomen noch Heteroatome, wie N, S, O und/oder P, im aromatischen Ringsystem tragen, wie z.B.
Pyridinyl, Triazinyl, Furyl, Thienyl, Thiazolyl, Thiazinyl, Pyrrolyl, Chinolinyl, die ihrerseits wiederum durch die obengenannten Substituenten substituiert sein können.

Insbesondere kommen solche p-Phenylendiamine der Formel (I) in Betracht, in denen

$R^1$ bis $R^4$ für Wasserstoff, Methyl, Ethyl, Propyl, t-Butyl, 2-Propyl, 2-Butyl, Methoxy, Ethoxy, Cyclohexyl, Benzoyl, Phenyl, Naphthyl, Chlorphenyl oder Toluyl
und

$R^5$ für Wasserstoff, 2-Propyl, 1,3-Dimethylbutyl oder Cyclohexyl stehen.

Als sterisch gehinderte Phenole kommen solche der allgemeinen Formel (II) in Betracht

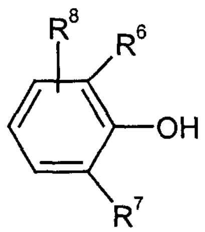

(II),

in denen

$R^6$ und $R^7$ gleich oder verschieden sind und für Wasserstoff, geradkettiges oder verzweigtes $C_1$-$C_{12}$-Alkyl, für verbrückendes $C_1$-$C_{12}$-Alkenyl sowie für Di(cyclopentadien)diyl stehen,
und

$R^8$ die Bedeutung von $R^6$ oder $R^7$ besitzt oder für $C_6$-$C_{12}$-Arylthio, verzweigtes oder geradkettiges $C_1$-$C_{12}$-Alkylthio oder für eine Gruppierung der Formel

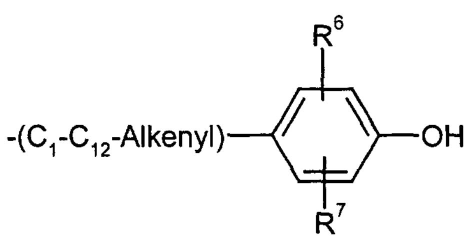

steht, mit der erwähnten Bedeutung von $R^6$ oder $R^7$.
Bevorzugt werden solche sterisch gehinderten Phenole eingesetzt, in denen

$R^6$ und $R^7$ für Wasserstoff, Methyl, Ethyl, 2-Propyl, tert.-Butyl, 1,1-Dimethylpropyl, Cyclohexyl, Cyclopentyl, Methylen, Ethylen, Butylen oder iso-Butylen stehen,
und

$R^8$ Wasserstoff, Methyl, tert.-Butyl, 2-Propyl, 2-Butyl, Cyclohexyl, Cyclopentyl, Nonylthio, Dodecylthio oder Cyclohexylthio bedeutet.

Die für die Herstellung der erfindungsgemäßen Alterungsschutzmittel einzusetzenden p-Phenylendiamine und die sterisch gehinderten Phenole sind dem Fachmann bekannt und beispielsweise beschrieben in J. G. Gillick, Elastomerics, 120 (8), (1988), 17-19, K. B. Chakraborty, G. Scott, J. Rekers, Polym. Prepr. (Am. Chem.

Soc., Div. Polym. Chem.) 26 (2), (1985), 31, J. A. Kuczkowski, J. G. Gillick, Rubber Chem. Technol. 57 (3), (1984), 621-651, G. Scott, Polym. Prepr. (Am. Chem. Soc., Div. Polym. Chem.), 25 (1), (1984), 62-63 und G. Scott, Gummi, Asbest, Kunstst. 31 (12), (1978), 934-938, 940, 966.

Als bifunktionelle Alkyl-, Aryl- und/oder Arylverbindungen kommen solche der allgemeinen Formeln (III), (IV) oder (V)

$$(F_1)_n\text{-Alkandiyl-}(F_2)_m \qquad \text{(III)}$$

$$(F_1)_n\text{-Aralkandiyl-}(F_2)_m \qquad \text{(IV)}$$

$$(F_1)_n\text{-Arendiyl-}(F_2)_m \qquad \text{(V)}$$

in Betracht, in denen

$F_1$ für Chlor, Brom, Iod, Hydroxyl, Carbonyl, Carboxyl, Olefin, Alkin, Sulfat, Sulfonat, Phosphat, Carbonat, Isocyanat oder Isothiocyanat steht,

$F_2$ für Halogen, Olefin, Alkin, Phosphat und Thiophosphat, Hydrogensulfid, Diund Trisulfan sowie Sulfit und Thiosulfat steht,

wobei

die Alkandiylgruppe 1 bis 30 Kohlenstoffatome besitzt, gegebenenfalls durch Heteroatome, wie Sauerstoff, Stickstoff oder Schwefel, ein- oder mehrfach unterbrochen, gegebenenfalls durch $C_1$-$C_{12}$-Alkyl, $C_1$-$C_{12}$-Alkyl-oxy, $C_1$-$C_{12}$-Alkyl-thio, $C_1$-$C_{12}$-Alkyl-amino, Di-($C_1$-$C_{12}$-alkyl)-amino, Benzyl, Phenyl oder $C_5$-$C_{12}$-Cycloalkyl substituiert und geradkettig, verzweigt oder cyclisch sein kann,

die Aralkandiylgruppe sowie die Arendiylgruppe 1 bis 30 Kohlenstoffatome besitzen und gegebenenfalls durch $C_1$-$C_{12}$-Alkyl, $C_1$-$C_{12}$-Alkyl-oxy, $C_1$-$C_{12}$-Alkyl-thio, $C_1C_{12}$-Alkyl-amino, Di-($C_1$-$C_{12}$-alkyl)-amino, Benzyl, 1,1-Dimethylbenzyl oder Phenyl substituiert sein können und ein oder mehrere Heteroatome der vorgenannten Art enthalten können
und

die Indices n und m gleich oder verschieden sind, wobei $1 \leq n \leq 10$ und $1 \leq m \leq 10$ ist.

Bevorzugt sind $1 \leq n \leq 6$ und $1 \leq m \leq 4$, besonders bevorzugt sind $1 \leq n \leq 3$ und $1 \leq m \leq 3$.

Die Reste $F^1$ und $F^2$ der Formeln (III) bis (V) können gleich oder verschieden sein.

Als funktionelle Gruppe $F_1$ wird bevorzugt genannt: Chlor, Brom, Hydroxy, Olefin, Carbaldehyd, Keton, $C_2$-$C_{30}$-Carboxylat- und deren Derivate, wie $C_2$-$C_{30}$-Carbon-säure-halogenide, -anhydride, -ester, -amide, Isocyanat, Sulfat und Sulfonat,
als funktionelle Gruppe $F_2$ kommt bevorzugt in Betracht: Chlor, Brom, Vinyl, Allyl, Styryl, Butandienyl, Cyclopentenyl, Cyclohexenyl, Cyclooctenyl, Cyclooctadienyl, Alkinyl, Hydrogensulfid sowie Di- und Trisulfan.

Als Alkandiylgruppen werden bevorzugt genannt: Methylen, $C_2$-$C_{30}$-Alkandiyl, $C_5$-$C_{20}$-Cycloalkandiyl, $C_6$-$C_{30}$-Bi-, tri- und tetracycloalkandiyl,

als Aralkandiylgruppe: durch gegebenenfalls geradkettige oder verzweigte $C_1$-$C_{12}$-Alkylgruppen einfach oder mehrfach substituiertes Phenyl oder Benzyl,

als Arendiylgruppe: durch gegebenenfalls geradkettige oder verzweigte $C_1$-$C_{12}$-Alkylgruppen einfach oder mehrfach substituiertes Phenylen, Naphthylen, Triazinylen, Pyrimidinylen.

Beispielsweise werden solche Verbindungen der Formeln (III) bis (V) bei der Herstellung der erfindungsgemäßen Alterungsschutzmittel eingesetzt, die nachfolgend genannt werden: $C_2$-$C_{30}$-Dihalogenalkane, $C_2$-$C_{30}$-Halogenalkene und -alkine, $C_2$-$C_{30}$-Halogencarbaldehyde, $C_2$-$C_{30}$-Halogenketone sowie $C_2$-$C_{30}$-Halogencarbonsäuren, einfach oder mehrfach ungesättigte $C_3$-$C_{30}$-Carbaldehyde, $C_3$-$C_{30}$-Ketone oder $C_3$-$C_{30}$-Carbonsäuren und deren $C_1$-$C_{12}$-Alkylester, $C_1$-$C_{12}$-Alkylamide, Anhydride und Säurehalogenide.

Insbesondere werden eingesetzt: Allylchlorid, 1,4-Dichlor-2-buten, 2,3-Dichlor-1-buten, 3,7-Dichlorcycloocta-1,5-dien, 3-Cyclohexencarbaldehyd, Isophoron, Phoron, Mesityloxid, Acrylsäure, Methacrylsäure, Crotonsäure, Maleinsäure, Fumarsäure, Ölsäure, Linolsäure, Linolensäure sowie deren $C_1$-$C_{12}$-Alkylester wie Methyl-, Ethyl-, Propyl-, 2-Propyl-, Butyl-, 2-Butyl-, tert.-Butyl, 2-Ethylhexyl-, Octyl- oder Dodecylester, sowie deren $C_1$-$C_{12}$-Alkylamide, wie N-Methyl-, N-Ethyl-, N-Propyl-, N-2-Propyl-, N-Butyl-, N-2-Butyl-, N-tert.-Butyl, N-2-Ethylhexyl-, N-Octyl- oder N-Dodecylcarbamid, sowie die entsprechenden $C_2$-$C_{24}$-Dialkylamide mit den benannten $C_1$-$C_{12}$-Alkylamidresten, die dabei gleich oder verschieden sein können.

Selbstverständlich können die oben genannten, gegebenenfalls substituierten Verbindungen auch im Gemisch miteinander eingesetzt werden.

Erfindungsgemäß werden pro Mol gegebenenfalls substituierter p-Phenylendiamine und/oder sterisch gehinderter Phenole 0,1 bis 4, bevorzugt 0,5 bis 3, insbesondere 1 bis 2 Mol bifunktioneller Alkyl- und/oder Aralkyl- und/oder Arylverbindungen der Formeln (III), (IV), (V) eingesetzt.

Zur Herstellung der erfindungsgemäßen covulkanisierbaren Alterungsschutzmittel wird das auf die oben geschilderte Weise erhaltene Umsetzungsprodukt anschließend weiter mit Schwefel und/oder schwefelliefernden Verbindungen zur Reaktion gebracht.

Als schwefelliefernde Verbindungen können zur Herstellung der erfindungsgemäßen Alterungsschutzmittel solche Verbindungen eingesetzt werden, die in der Lage sind, bei der Reaktion Schwefel freizusetzen. Diese Schwefelspender sind dem Fachmann bekannt (siehe z.B. Werner Hofmann, "Kautschuktechnologie", Genter Verlag, Stuttgart 1980, 256-258). Zu diesem Zweck sind Verbindungen geeignet, die eine oder mehrere direkte Schwefel-Schwefel-Bindungen enthalten, wie beispielsweise $C_1$-$C_{30}$-Alkyldi-, -tri-, -tetra-, -penta- und -polysulfide oder Di($C_1$-$C_{30}$-Alkylamino)-N-di-, N-tri-, -N-tetra- und N-polysulfide.

Der Schwefel und/oder die schwefelliefernden Verbindungen werden dem erhaltenen Umsetzungsprodukt in einer solchen Menge zugegeben, dass pro funktionelle Gruppe $F_2$ 1 bis 8 Mol, bevorzugt 2 bis 6 Mol, insbesondere 3 bis 5 Mol an Schwefel und/oder schwefelliefernde Verbindung eingesetzt werden. Die angegebenen, einzusetzenden Mole Schwefel definieren sich hierbei als 1/8 Mol $S_8$ (Cyclooctaschwefel).

Wie zuvor erwähnt, können die substituierten p-Phenylendiamine und die sterisch gehinderten Phenole im Gemisch untereinander eingesetzt werden. Das günstigste Mischungsverhältnis kann leicht durch Vorversuche ermittelt werden und richtet sich beispielsweise nach den geforderten physikalischen Eigenschaften des Alterungsschutzmittels. Das gleiche gilt für die gegebenenfalls einzusetzende Mischung an bifunktionellen Alkyl- und Aralkyl- und Arylverbindungen sowie für einzusetzende Mischungen von Schwefel und schwefelhaltigen Verbindungen.

Die Umsetzung der p-Phenylendiamine und/oder sterisch gehinderten Phenole mit den bifunktionellen Alkyl-, Aralkyl- oder Arylverbindungen wird üblicherweise in Gegenwart von inerten, organischen Lösungsmitteln durchgeführt.

Als inerte, organische Lösungsmittel kommen beispielsweise in Betracht: aliphatische oder aromatische Kohlenwasserstoffe, die gegebenenfalls substituiert sein können mit Alkyl-, Alkoxy-, Halogen-, Nitro-, Amino- oder Sulfogruppen, sowie aliphatische oder aromatische Ether, Amine und Sulfide.

Bevorzugt werden als Lösungsmittel eingesetzt: Alkylbenzole, Xylol und Benzine der für solche Zwecke bekannten Art.

Selbstverständlich kann die erwähnte Umsetzung auch ohne Lösungsmittel durchgeführt werden, beispielsweise in der Schmelze oder in einem Überschuss an in flüssiger Form vorliegenden bifunktionellen Verbindungen der Formeln (III), (IV), (V).

Die günstigste Menge an einzusetzendem Lösungsmittel kann leicht durch entsprechende Vorversuche ermittelt werden.

Üblicherweise wird die erste Reaktionsstufe zur Herstellung des erfindungsgemäßen Alterungsschutzmittels

bei Temperaturen von -20 bis +200°C, bevorzugt bei 40 bis 140°C durchgeführt.

Falls die erste Reaktionsstufe mit einem Überschuss an bifünktionellen Alkyl-, Arylund/oder Aralkylverbindungen durchgeführt wurde, wird der Überschuss an diesen Verbindungen vor der weiteren Umsetzung mit Schwefel und/oder schwefelhaltigen bzw. schwefelliefernde Verbindungen abdestilliert. Bei Einsatz eines inerten, organischen Lösungsmittels kann dieses im Reaktionsprodukt verbleiben.

Das in der ersten Reaktionsstufe erhaltene Umsetzungsprodukt wird bei Temperaturen von ca. 40 bis 200°C, bevorzugt 110 bis 160°C, besonders bevorzugt bei 130 bis 150°C, mit Schwefel und/oder den schwefelhaltigen Verbindungen zur Reaktion gebracht.

Selbstverständlich kann die Herstellung des erfindungsgemäßen Alterungsschutzmittels durch die zuvor beschriebenen Umsetzungen durch geeignete Katalysatoren beschleunigt werden. Als geeignete Katalysatoren für die erste Reaktionsstufe kommen beispielsweise in Betracht: Lewissäuren, wie z.B. Aluminium-, Zink-, Zinn-, Titan-, Eisen- oder Borhalogenide, Brönstedtsäuren, wie z.B. Schwefel- und Sulfonsäuren, Salzsäure, Phosphorsäure, oder auch Basen, wie Amine oder Metallhydroxide, beispielsweise Natrium-, Kalium- und Kalziumhydroxid und deren wässrige Lösungen, wie sie für solche Zwecke bekannt sind.

Als geeignete Katalysatoren für die zweite Reaktionsstufe kommen beispielsweise in Betracht: Ammoniak, $C_1$-$C_{36}$-Alkylamine, $C_2$-$C_{40}$-Dialkylamine und deren Ammoniumsalze, Schwefelwasserstoff, Di-, Tri- und Tetrasulfan sowie deren $C_1$-$C_{36}$-Alkyl oder $C_1$-$C_{40}$-Dialkylderivate, weiterhin Salze von Metallen der Gruppen 1, 2, 12, mit $C_1$-$C_{36}$-Dithiocarbonsäuren und deren ($C_1$-$C_{36}$-Alkyl)-Amiden, wie beispielsweise $C_1$-$C_{36}$-Alkyldithiocarbonaten, $C_1$-$C_{36}$-Alkyldithiocarbamaten, ($C_1$-$C_{36}$-Alkyl)-Mercaptothiazolen oder ($C_1$-$C_{36}$-Alkyl)-Mercaptobenzothiazolen. Weitere Katalysatoren sind Salze von Metallen der Gruppen 1, 2, 12 mit Thioschwefelsäuren und Thiophosphorsäuren, Schwefelwasserstoff, Di-, Tri- und Tetrasulfan, mit Selen-, Tellur-, Phosphor-, Cyan- und Iodwasserstoffsäure.

Die Katalysatoren werden in den üblichen Mengen (0,1 bis 10 mol-%, bezogen auf ein mol bifunktioneller Verbindungen der Formeln (III), (IV) oder (V) eingesetzt.

Das erfindungsgemäß erhaltene Alterungsschutzmittel wird wie erwähnt zum Schutz von Kautschukvulkanisaten eingesetzt, die störenden Umwelteinflüssen ausgesetzt sind. Selbstverständlich ist es möglich, das Alterungsschutzmittel mit den zum Schutz von Kautschukvulkanisaten dem Fachmann bekannten Alterungsschutzmitteln zu kombinieren. Das günstigste Mischungsverhältnis kann dabei leicht durch entsprechende Vorversuche ermittelt werden und richtet sich nach dem jeweiligen Verwendungszweck des zu schützenden Vulkanisats.

Darüber hinaus ist es möglich, das erfindungsgemäße Alterungsschutzmittel mit einem der bekannten Ozonschutzmittel abzumischen, um einen verbesserten Ozonschutz der Kautschukvulkanisate zu erhalten. Auch hierbei kann der Fachmann das günstigste Mischungsverhältnis je nach dem Verwendungszweck des Kautschukvulkanisats leicht durch Vorversuche ermitteln.

Das erfindungsgemäße Alterungsschutzmittel wird üblicherweise in Mengen von 0,5 Gew.-% bis 10 Gew.-%, bevorzugt 2 Gew.-% bis 5 Gew.-%, bezogen auf 100 Teile des zu schützenden Kautschuks, eingesetzt.

Die Kautschukmischung kann selbstverständlich noch weitere Kautschukhilfsprodukte enthalten, wie Reaktionsbeschleuniger, Wärmestabilisatoren, Lichtschutzmittel, Verarbeitungshilfsmittel, Weichmacher, Tackifier, Treibmittel, Farbstoffe, Pigmente, Wachse, Streckmittel, organische Säuren, Verzögerer, Metalloxide sowie Aktivatoren wie Triethanolamin, Polyethylenglykol, Hexantriol, die in der Gummiindustrie bekannt und üblich sind. Die Kautschukhilfsmittel werden in üblichen Mengen zugemischt und richten sich nach dem jeweils beabsichtigten Verwendungszweck. Übliche Mengen sind beispielsweise Mengen von 0,1 bis 50 Gew.-%, bezogen auf die Gesamtmenge des eingesetzten Kautschuks.

Neben den zuvor erwähnten Hilfsprodukten können den Kautschukmischungen die bekannten Vernetzer zugegeben werden, wie Schwefel oder Schwefelspender, und Vulkanisationsbeschleuniger, wie Mercaptobenzthiazole, Benzthiazolsulfenamide, Guanidine, Thiurame, Dithiocarbamate, Thioharnstoffe und/oder Thiocarbonate. Die Vulkanisationsbeschleuniger und die erwähnten Vernetzer werden üblicherweise in Mengen von 0,1 bis 10 Gew.-%, bevorzugt 0,5 bis 4 Gew.-%, bezogen auf die Gesamtmenge des jeweils eingesetzten Kautschuks, eingesetzt.

Die Vulkanisation der Kautschukmischungen, enthaltend die erfindungsgemäßen Alterungsschutzmittel, kann bei üblichen Temperaturen von 100 bis 200°C, bevorzugt 130 bis 180°C (gegebenenfalls unter 10 bis 200 bar Druck), erfolgen.

Die weitere Abmischung der Kautschuke mit den anderen erwähnten Kautschukhilfsprodukten, Vernetzern und Beschleunigern kann in üblicher Weise mit Hilfe von geeigneten Mischaggregaten, wie Walzen, Innenmischer und Mischextruder, durchgeführt werden.

Die erhaltenen Kautschukmischungen können gegebenenfalls auf übliche Weise compoundiert und vulkanisiert werden, wie beispielsweise näher in Encyclopedia of Polymer Science and Engineering, Vol. 4, S. 66 ff(Compoundierung) und Vol. 17, S. 666 ff (Vulkanisation) beschrieben wird.

**Beispiele**

Herstellung der erfindungsgemäßen covulkanisierbaren Alterungsschutzmittel

Verbindung A

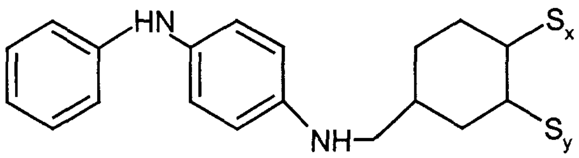

x+y = 1 bis 10

In einem 2 l Vierhalskolben mit Wasserabscheider wurden 440 g (4 mol) 3-Cyclohexencarbaldehyd in 900 ml Hexan gelöst und unter Rühren zum Rückfluss erhitzt. 368 g (2 mol) 4-ADPA (4-Aminodiphenylamin) wurden zugesetzt, wobei sich innerhalb von 4,5 h 34,5 ml Reaktionswasser bildeten. Nach dem Abdestillieren von Lösungsmittel und überschüssigem Aldehyd wurde der Rückstand bei 50°C in 500 ml Methanol aufgenommen, portionsweise in 3,5 h mit 110 g (3,0 mol) Natriumborhydrid versetzt und weitere 6 h bei 60°C gerührt. Nach Abdestillieren des Lösungsmittels wurden zu dem Rohprodukt 600 ml Toluol und 500 ml Wasser gegeben, die organische Phase noch zweimal mit 500 ml Wasser gewaschen und über 50 g Natriumsulfat filtriert. Das Lösungsmittel wurde im Wasserstrahlvakuum (20 mbar) bei 80°C abdestilliert und das Produkt mittels 15 cm Vigreuxkolonne rektifiziert. Es wurde 300 g wachsartiger Feststoff mit einem Siedepunkt von 219°C/0,2 mbar erhalten.

83,7 g (0,3 mol) dieses Rohproduktes (N-Phenyl-N'-(3-cyclohexenyl)methyl-p-phenylendiamin), 38,4 g (1,2 mol) Schwefel und 200 ml Xylol wurden unter Rühren während 11 h zum Rückfluss erhitzt. Das Xylol wurde abdestilliert und der Rückstand, 119,6 g schwarzer Feststoff, isoliert.
Elementaranalyse: S: 28,2 %, H: 5,4 %, C: 58,5 %, N: 7,6 %

Verbindung B

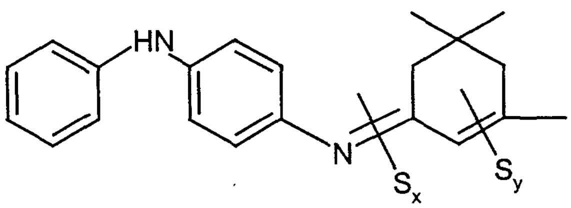

x+y = 1 bis 10

In einem 1l Vierhalskolben mit Wasserabscheider und Thermometer wurden 1844 g (1 mol) 4-ADPA (4-Aminodiphenylamin) in 600 ml Xylol unter Rühren gelöst und mit 2 g p-Toluolsulfonsäure zum Rückfluss erhitzt und 179,7 g (1,3 mol) Isophoron zudosiert. Innerhalb von 8 h spalteten sich 16,5 ml Wasser ab. Anschließend wurden dem Reaktionsgemisch 500 ml Wasser und 50 g $NaHCO_3$ zugesetzt, gerührt, die Phasen im Scheidetrichter getrennt und die organische Phase über 50 g $Na_2SO_4$ filtriert. Nach Destillation (0,2 mbar, 100°C) der flüchtigen Anteile wurde das Zwischenprodukt (schwarz-brauner Rückstand) aus 600 ml Toluol und 300 ml n-Hexan umkristallisiert. Ausbeute: 99 g gelbes, kristallines N-Phenyl-N'-3,3,5-trimethylcyclohex-2-enylen-p-phenylendiamin, Fp.: 126-128°C.

15,3 g (0,05 mol) des obigen Reaktionsproduktes und 7,4 g (0,23 mol) Schwefel wurden in 100 ml Xylol unter Rühren gelöst und 5 h bei 140°C gerührt, bis die DC-Kontrolle fast vollständigen Umsatz zeigte. Nach Abziehen des Lösungsmittels (70°C, 0,2 mbar) wurden 19,8 g schwarzes, viskoses Produkt erhalten.

Verbindung C

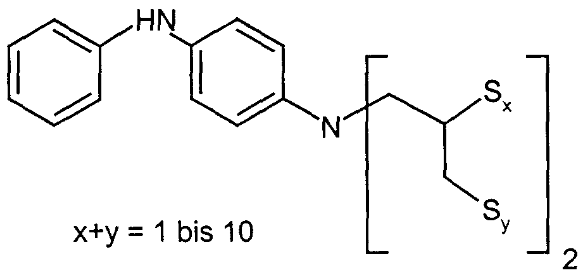

x+y = 1 bis 10

Zu einer Lösung von 368 g (2 mol) 4-ADPA und 445,2 g (4,4 mol) Triethylamin in 600 ml Toluol wurden bei Siedetemperatur innerhalb von 2 Stunden 306 g (4 mol) Allylchlorid zugetropft und das Gemisch noch 6 h am Rückfluss gehalten, wobei ein Niederschlag entstand. Nach Zusatz von 400 ml Toluol und 1l Wasser wurden die Phasen getrennt. Die organische Phase wurde noch zweimal mit je 500 ml Wasser gewaschen und mit 50 g Natriumsulfat getrocknet. Nach destillativer Entfernung des Lösungsmittels wurde das Produkt im Vakuum rektifiziert: 435 g eines gelben Öls, Siedepunkt: 160°C / 0,15 mbar.

Zu diesem Zwischenprodukt, 435 g (1.645 mol), sind in 500 ml Xylol bei 130°C innerhalb von 2 h portionsweise 211 g (6,58 mol) Schwefel zugesetzt worden. Nach weiteren 13 h bei 130 - 140°C wurde das Reaktionsgemisch abgekühlt, das Lösungsmittel im Vakuum entfernt und der Rückstand isoliert: 573,7 g dunkler Feststoff. Elementaranalyse: C: 60,7 %, H: 5,2 %, N: 8,3 %, S: 26,1 %

Verbindung D

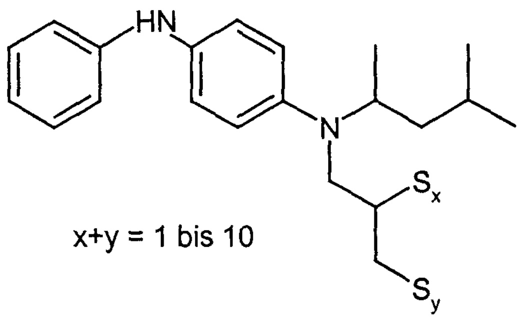

x+y = 1 bis 10

Zu einer Lösung von 402 g (1,5 mol) 6PPD (4-(1,3-Dimethylbutylamino)-diphenylamin) und 401 g (4 mol) Triethylamin in 800 ml Toluol wurden unter Rühren bei Rückflusstemperatur innerhalb von 2,5 h 246 g (3,6 mol) Allylchlorid zugetropft und dieses Gemisch weitere 12 h zum Sieden erhitzt. Das Reaktionsgemisch wurde mit 200 ml Toluol und 1l Wasser versetzt, gerührt, die organische Phase noch zweimal mit je 1l Wasser gewaschen, die Phasen getrennt und die organische Phase über 50 g $Na_2SO_4$ getrocknet. Destillativ wurde das Lösungsmittel entfernt und das Produkt über eine kleine Vigreux-Kolonne rektifiziert. Es wurden 385 g eines gelbbraunen, öligen Produktgemisches erhalten. Siedepunkt: 163-166°C/0,1 mbar. Das Rohprodukt, 385 g (1,14 mol), wurde auf 130°C erhitzt und unter Rühren innerhalb von 4,5 h portionsweise mit 218,9 g (6,84 mol) Schwefel versetzt. Nach 12 h bei 135 bis 140°C wurde das Reaktionsgemisch auf Raumtemperatur abgekühlt. Es wurden 589 g eines in der Kälte hochviskosen, dunklen Produktes erhalten.

Verbindung E

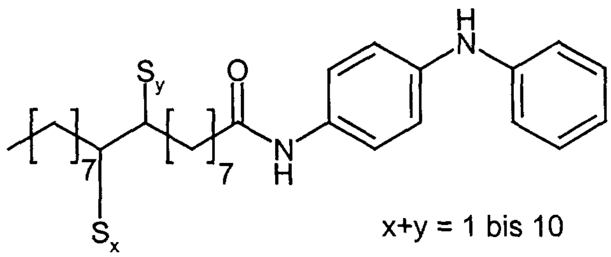

148 g (0,5 mol) Ölsäuremethylester, 55,2 g (0,3 mol) 4-ADPA und 2 g Natriummethylat wurden unter Rühren bei 180°C über eine Zeitspanne von 9 h zur Reaktion gebracht, wobei 9 ml Methanol abgespalten und abdestilliert wurden. Der Überschuss an Ölsäuremethylester wurde im Hochvakuum abdestilliert (230°C, 0,2 mbar). Der Destillationsrückstand ist in 500 ml Toluol und 500 ml Wasser aufgenommen und ausgeschüttelt worden. Nach Trennung der Phasen im Scheidetrichter wurde die organische Phase über 30 g Natriumsulfat getrocknet. Anschließend wurde das Toluol im Wasserstrahlvakuum (20 mbar, 100°C Badtemperatur) abdestilliert. Der Rückstand wurde isoliert: Ausbeute: 116 g eines braunen Wachses.

Das Reaktionsprodukt wurde in 500 ml Xylol gelöst, mit 24,7 g Schwefel versetzt und 3 h unter Rückfluss gehalten. Das Lösungsmittel wurde abdestilliert und der Rückstand isoliert: 120 g schwarzer Feststoff.

Verbindung F

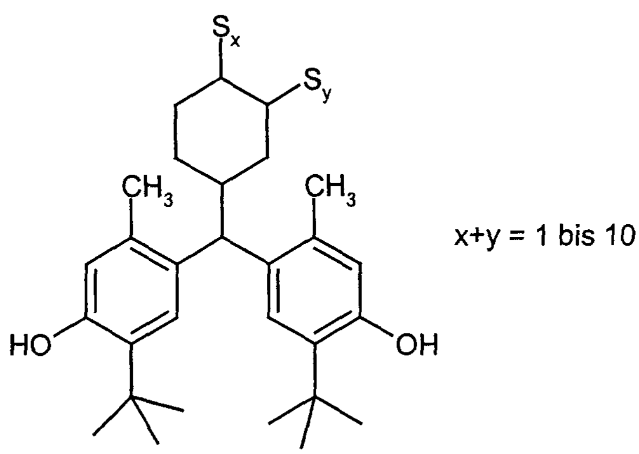

Ein Gemisch aus 669,0 g (3 mol) 6-tert. Butyl-3-methylphenol, 360 ml Benzin und 3,58 g konz. Schwefelsäure wurden unter Rühren auf 85°C erhitzt. Eine Lösung von 165 g (1,5 mol) 3-Cyclohexencarbaldehyd in 80 ml Benzin wurde innerhalb von 5 h zugegeben. Währenddessen wurden am Wasserabscheider 27 ml Wasser abgeschieden. Nach Filtration der Suspension wurde der Filterrückstand getrocknet. Ausbeute: 341 g (0,812 mol) eines hellgrauen Feststoffes. Schmelzpunkt: 237 - 244°C.

Vom obigen Reaktionsprodukt (3-Cyclohexenylmethylen-4,4-bis(2-tert.-butyl-5-methylphenol) wurden 21 g (0,05 mol) in 20 ml Xylol bei 115°C gelöst, diese Lösung mit 9,6 g (0,30 mol) Schwefel versetzt und 49 h bei Rückfluss gerührt. Das Lösungsmittel wurde abdestilliert. Der Rückstand bestand aus 36 g eines braunen Feststoffes.

Ergebnisse der anwendungstechnischen Untersuchung:

| Mischungsrezeptur | |
|---|---|
| TSR 5 [1] | 100 |
| Renopal® 450 [2] | 6 |
| Stearinsäure [3] | 2 |
| Antilux® 111 [4] | 2 |
| Zinkweiß RS | 5 |
| Rhenocure® IS90/G [5] | 2,2 |
| Vulkacit® CZ [6] | 1,5 |
| Total: | 118,7 |

[1] TSR 5, Weber & Schaer GmbH & Co
[2] Renopal 450, Fuchs Mineralölwerke GmbH
[3] Stearinsäure, Henkel KgaA Dehydag Oleogrundstoffe
[4] Antilux 111, Rhein-Chemie Rheinau GmbH
[5] Rhenocure® IS90/G, Rhein-Chemie Rheinau GmbH
[6] Vulkacit® CZ, Bayer AG

| | phr: parts per hundred rubber | | | | | |
|---|---|---|---|---|---|---|
| Masterbatch: | 118,7 | 118,7 | 118,7 | 118,7 | 118,7 | 118,7 |
| 6PPD | - | 4 | - | - | - | - |
| A | - | - | 4 | - | - | - |
| B | - | - | - | 4 | - | - |
| C | - | - | - | - | 4 | - |
| D | - | - | - | - | - | 4 |
| Walze: | | | | | | |
| Rhenocure IS 90G | 2,2 | 2,2 | 2,2 | 2,2 | 2,2 | 2,2 |
| Vulkacit CZ/C | 1,5 | 1,5 | 1,5 | 1,5 | 1,5 | 1,5 |
| Probekörper | Nullprobe | 6PPD | A | B | C | D |

Die Kautschukmischungen wurden wie folgt hergestellt: In einem TPE-Kneter GK 1,5 E (Volumen ca. 1500ml) Temperatur = 40°C wurden alle angeführten Substanzen, außer Rhenocure® IS90/G und Vulkacit® CZ in die Kautschukmatrix eingemischt. Anschließend wurden Rhenocure® IS90/G und Vulkacit® CZ auf einer Walze bei 40°C in die Mischung eingebracht. Die Drehzahl der Walzen betrug = 12 Um/min, Friktion = 1,22.

Die Vulkanisation der Mischungen zu Gummiplatten (100 x 100 x 2 mm) erfolgte danach in elektrischen Heizpressen (300 bar) bei 150°C bis T 90 + 5 min. der Rheometerkurven.

Extraktionsversuche

Die Vulkanisate wurden in saurem Wasser bei pH 4 und 40°C gelagert. Nach einer Immersionsdauer von 7 und von 28 Tagen wurden die Proben entnommen und der darin enthaltene Gesamtstickstoffgehalt nach Kjeldahl bestimmt. Der Stickstoffgehalt steht stellvertretend für die Summe aus zugesetzten, aminischen erfindungsgemäßen Alterungsschutzmitteln bzw. 6PPD und den üblichen, aminhaltigen Vulkanisationsbeschleunigern.

Ein Vergleich der Gehalte an Stickstoff zeigt deutlich, dass ein konventionelles Alterungsschutzmittel, wie beispielsweise Vulkanox® 4020, nach vierwöchigem Wasserkontakt bereits in deutlicher Menge aus dem Vulkanisat extrahiert wurde.

Überraschend anders hingegen verhalten sich Vulkanisate, die die erfindungsgemäßen Alterungsschutzmittel (Verbindungen A bis D) enthalten.

Nach anfänglichem Verlust an Stickstoff, der etwa dem normalen Verlust der Nullprobe entspricht, verlieren die Vulkanisate, enthaltend die Verbindungen A bis D, kaum noch Stickstoff bzw. Amin.

Die im Vulkanisat enthaltene Menge an aminischem Alterungsschutzmittel verringert sich durch die Lagerung in saurem Wasser kaum.

Auch nach längerem Kontakt mit extraktiven Medien, wie z.B. saurem Wasser, bleibt ein überwiegender Teil an zugesetztem, aminischen erfindungsgemäßen Alterungsschutzmittel erhalten. Parallel dazu bleibt der Schutz des Vulkanisats vor Alterungserscheinungen erhalten.

| Probekörper | N-Gehalt [%] | | |
|---|---|---|---|
| | 0 Tage | 7 Tage | 28 Tage |
| | Extraktionsdauer (Wasser) | Extraktionsdauer | Extraktionsdauer |
| Nullprobe | 0,53 | 0,52 | 0,51 |
| 6PPD | 0,68 | 0,59 | 0,54 |
| A | 0,63 | 0,59 | 0,58 |
| B | 0,65 | 0,59 | 0,59 |
| C | 0,65 | 0,59 | 0,59 |
| D | 0,68 | 0,61 | 0,6 |

**Patentansprüche**

1. Covulkanisierbare Alterungsschutzmittel, herstellbar durch Umsetzung von gegebenenfalls substituierten p-Phenylendiaminen und/oder sterisch gehinderten Phenolen mit bifunktionellen Alkyl-, Aryl- und/oder Aralkylverbindungen und anschließender Reaktion des so erhaltenen Produkts mit Schwefel und/oder schwefelliefernden Verbindungen.

2. Covulkanisierbare Alterungsschutzmittel nach Anspruch 1, **dadurch gekennzeichnet, dass** als p-Phenylendiamine solche der Formel (I)

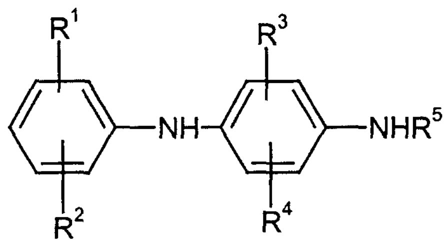

(I),

in der

$R^1$ bis $R^4$ gleich oder verschieden sind und für Wasserstoff, geradkettiges oder verzweigtes $C_1$-$C_{12}$-Alkyl, $C_1$-$C_{12}$-Alkoxy, $C_1$-$C_{12}$-Alkyl-thio, $C_1$-$C_{12}$-Alkyl-amino, Di-($C_1$-$C_{12}$-alkyl)-amino, Benzyl, 1,1-Dimethylbenzyl oder Phenyl stehen
und

$R^5$ für Wasserstoff, Phenyl, $C_6$-$C_{12}$-Aryl, $C_1$-$C_{12}$-Heteroaryl oder $C_1$-$C_{12}$-Alkyl steht.

3. Covulkanisierbare Alterungsschutzmittel nach Anspruch 1, **dadurch gekennzeichnet, dass** als sterisch gehinderte Phenole solche der Formel (II)

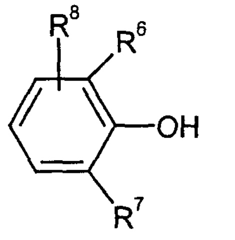

(II),

eingesetzt werden,

in denen

$R^6$und $R^7$ gleich oder verschieden sind und für Wasserstoff, geradkettiges oder verzweigtes $C_1$-$C_{12}$-Alkyl, für verbrückendes $C_1$-$C_{12}$-Alkenyl, sowie für Di(cyclopentadien)diyl stehen, und

$R^8$ die Bedeutung von $R^6$ oder $R^7$ besitzt oder für $C_6$-$C_{12}$-Arylthio, verzeigtes oder geradkettiges $C_1$-$C_{12}$-Alkylthio oder für eine Gruppierung der Formel

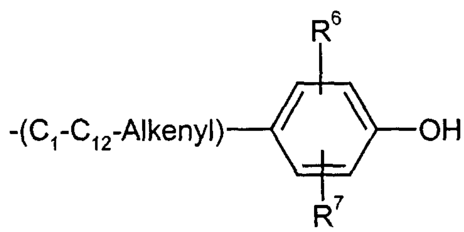

steht, mit der erwähnten Bedeutung von $R^6$ oder $R^7$.

**4.** Covulkanisierbare Alterungsschutzmittel nach Anspruch 1, **dadurch gekennzeichnet, dass** als bifunktionelle Alkyl-, Aryl- und/oder Aralkylverbindungen solche der Formeln (III), (IV) und (V) eingesetzt werden

$$(F_1)_n\text{-Alkandiyl-}(F_2)_m \qquad \text{(III)}$$

$$(F_1)_n\text{-Aralkandiyl-}(F_2)_m \qquad \text{(IV)}$$

$$(F_1)_n\text{-Arendiyl-}(F_2)_m\ , \qquad \text{(V)}$$

in denen

$F_1$ für Chlor, Brom, Iod, Hydroxyl, Carbonyl, Carboxyl, Olefin, Alkin, Sulfat, Sulfonat, Phosphat, Carbonat, Isocyanat oder Isothiocyanat steht, und

$F_2$ für Halogen, Olefin, Alkin, Phosphat und Thiophosphat, Hydrogensulfid, Di- und Trisulfan sowie Sulfit und Thiosulfat steht, wobei

die Alkandiylgruppe 1 bis 30 Kohlenstoffatome besitzt, gegebenenfalls durch Heteroatome, ein- oder mehrfach unterbrochen, gegebenenfalls durch $C_1$-$C_{12}$-Alkyl, $C_1$-$C_{12}$-Alkyl-oxy, $C_1$-$C_{12}$-Alkyl-thio, $C_1$-$C_{12}$-Alkyl-amino, Di($C_1$-$C_{12}$-alkyl)-amino, Benzyl, Phenyl oder $C_5$-$C_{12}$-Cycloalkyl substituiert und geradkettig, verzweigt oder cyclisch sein kann,

die Aralkandiylgruppe sowie die Arendiylgruppe 1 bis 30 Kohlenstoffatome besitzen und gegebenenfalls durch $C_1$-$C_{12}$-Alkyl, $C_1$-$C_{12}$-Alkyl-oxy, $C_1$-$C_{12}$-Alkyl-thio, $C_1$-$C_{12}$-Alkyl-amino, Di-($C_1$-$C_{12}$-alkyl)-amino, Benzyl, 1,1-Dimethylbenzyl oder Phenyl substituiert sein können und ein oder mehrere Heteroatome enthalten können und

n und m gleich oder verschieden sind mit $1 \leq n \leq 10$ und $1 \leq m \leq 10$.

**5.** Covulkanisierbare Alterungsschutzmittel nach Anspruch 1, **dadurch gekennzeichnet, dass** pro mol gegebenenfalls substituierter p-Phenylendiamine und/oder sterisch gehinderter Phenole 0,1 bis 4 mol bifunktioneller Alkyl-, Aryl- und/oder Aralkylverbindungen der Formeln (III), (IV) oder (V) eingesetzt werden.

6. Covulkanisierbare Alterungsschutzmittel nach Anspruch 1, **dadurch gekennzeichnet, dass** der Schwefel und/oder die schwefelliefernden Verbindungen in solchen Mengen eingesetzt werden, dass pro fünktionelle Gruppe $F_2$ 1 bis 8 mol an Schwefel und/oder schwefelliefernden Verbindungen eingesetzt werden.

7. Verwendung des Alterungsschutzmittels nach Anspruch 1 zur Herstellung von Kautschukvulkanisaten.